# EUROPÄISCHE PATENTANMELDUNG

(11) **EP 2 484 421 A1**
(43) Veröffentlichungstag der Anmeldung: **08.08.2012**
(21) Anmeldenummer: 11153550.6
(22) Anmeldetag: 07.02.2011
(51) Int. Cl.: B01D 1/28, B01D 3/00, C07C 209/86

(54) **Verfahren zur Abtrennung von leichtsiedenden Komponenten aus einem Hexamethylendiamin enthaltenden Gemisch**

(71) Anmelder: BASF SE, 67056 Ludwigshafen (DE)
(72) Erfinder: Ottenbacher, Markus, 68809 Neulußheim (DE); Hütten, Frank, 68165 Mannheim (DE)

(57) **Zusammenfassung**

Verfahren zur Abtrennung von leichtsiedenden Komponenten aus einem Hexamethylendiamin enthaltenden Gemisch, wobei das Gemisch als Feedstrom (301, 401) einer Destillationsvorrichtung (300, 400) zugeführt wird, die einen Verstärkungsteil (310, 410) und einen Abtriebsteil (320, 420) umfasst, am Kopf des Verstärkungsteils ein an leichtsiedenden Komponenten angereicherter Kopfstrom abgezogen wird, am Sumpf des Abtriebsteils ein um leichtsiedende Komponenten abgereicherter Sumpfstrom (322, 422) abgezogen wird, von dem ein Teilstrom (321, 421) einem Sumpfverdampfer (330, 430) zugeführt, dort zumindest teilweise verdampft und in den Abtriebsteil zurückgeführt wird, dadurch gekennzeichnet, dass aus der Destillationsvorrichtung ein dampfförmiger Seitenstrom (360, 460) abgezogen und einem Verdichter (350, 450) zugeführt wird, der verdichtete Strom als Heizmedium zum Sumpfverdampfer (330, 430) geleitet wird, wo er durch Abgabe von Wärme den Teilstrom (321, 421) aus dem Sumpf zumindest teilweise verdampft, und der zumindest teilweise kondensierte Seitenstrom nach Austritt aus dem Sumpfverdampfer wieder der Destillationsvorrichtung zugeführt wird.

## Beschreibung

Die vorliegende Erfindung betrifft ein Verfahren zur Abtrennung von leichtsiedenden Komponenten aus einem Hexamethylendiamin enthaltenden Gemisch, wobei das Gemisch als Feedstrom einer Destillationsvorrichtung zugeführt wird, die einen Verstärkungsteil und einen Abtriebsteil umfasst, am Kopf des Verstärkungsteils ein an leichtsiedenden Komponenten angereicherter Kopfstrom abgezogen wird, und am Sumpf des Abtriebsteils ein um leichtsiedende Komponenten abgereicherter Sumpfstrom abgezogen wird, von dem ein Teilstrom einem Sumpfverdampfer zugeführt, dort zumindest teilweise verdampft und in den Abtriebsteil zurückgeführt wird.

Hexamethylendiamin stellt ein wichtiges Monomer zur Herstellung von Polyamiden dar. Aus Adipinsäure und Hexamethylendiamin wird durch Polykondensation Polyamid 6.6 hergestellt. Durch Umsetzung von Hexamethylendiamin mit Phosgen erhält man Diisocyanat, das als Komponente zur Herstellung von Polyurethan-Harzen und -Schaumstoffen dient.

Großtechnisch wird Hexamethylendiamin durch katalytische Flüssigphasen-Hydrierung von Adipodinitril hergestellt, wobei zwei unterschiedliche Verfahren und Katalysatorsysteme für die Hydrierung etabliert sind. Bei einem von der Firma DuPont entwickelten Hochdruckverfahren erfolgt die Hydrierung bei einer Temperatur von 100 bis 200°C und einem Druck von 200 bis 400 bar in Gegenwart von Eisen-Festbett-Katalysatoren und Ammoniak als Lösungsmittel. Bei einem von der Societa Rhodiatoce entwickelten Niederdruck-Suspensionsverfahren wird bei einer Temperatur von 60 bis 100°C und einem Druck von 20 bis 50 bar in Gegenwart eines mit Alkali modifizierten Raney-Nickel-Katalysators und Hexamethylendiamin als Lösungsmittel hydriert. Der Katalysator wird kontinuierlich ausgeschleust und durch Waschen mit Wasser regeneriert. Nach beiden Verfahren entsteht Hexamethylendiamin mit einer Selektivität von 99% bei einem Umsatz von ebenfalls 99%.

Die Aufarbeitung von Hexamethylendiamin erfolgt üblicherweise destillativ in einer Sequenz von Destillationskolonnen. In dem Buch "Winnacker/Küchler: Chemische Technik, Band 5, Organische Zwischenverbindungen, Polymere, 5. Auflage, Wiley-VCH-Verlag 2005, Seiten 242 und 244 und Abb. 9.2, Seite 243" beispielsweise ist die Aufarbeitung von Hexamethylendiamin beschrieben, das nach dem Niederdruck-Suspensionsverfahren hergestellt wurde. Sie wird nach Abtrennung des Raney-Nickel-Katalysators in vier Destillationskolonnen kontinuierlich durchgeführt. In der ersten Kolonne wird Wasser über Kopf abdestilliert. Das Sumpfprodukt der ersten Kolonne wird in einem zweiten Schritt über Sumpf von Hochsiedern befreit. Das Kopfprodukt des zweiten Trennschritts wird gasförmig in eine dritte Kolonne geleitet, in der Leichtsieder über Kopf ausgeschleust werden. Das Sumpfprodukt der dritten Kolonne wird auf eine vierte Kolonne gegeben. Als Kopfprodukt werden Leichtsieder und als Sumpfprodukt Hochsieder abgetrennt. Aus einem Seitenabzug der vierten Kolonne wird Hexamethylendiamin mit hoher Reinheit entnommen.

Insbesondere für die Herstellung von Polyamid 6.6 werden an den Einsatzstoff Hexamethylendiamin hohe Reinheitsanforderungen gestellt. Die im Roh-Hexamethylendiamin enthaltenen Nebenprodukte cis- und trans-1.2-Diaminocyclohexan, cis- und trans-2-Aminomethylcyclopentylamin, 6-Aminocapronitril, Hexamethylenimin, Tetrahydroazepin, Diaminomethylpentan und Ammoniak müssen im Rein-Hexamethylendiamin jeweils auf Werte deutlich unter 100 ppm abgereichert sein. Andernfalls kann sich das aus dem zu stark verunreinigten Hexamethylendiamin hergestellte Polyamid 6.6 verfärben.

Die destillative Reinigung des Hexamethylendiamins, bei der einige Nebenprodukte wie cis- und trans-1.2-Diaminocyclohexan sowie insbesondere cis- und trans-2-Aminomethylcyclopentylamin abgetrennt werden, erfordert eine hohe Zahl an theoretischen Böden bei gleichzeitig hohem Rücklaufverhältnis. Aus der Patentschrift EP 0931054 B1 ist bekannt, für die destillative Abtrennung der Leichtsieder druckverlustarme Packungen zu verwenden und die Destillation im Vakuum zu betreiben. Um Hexamethylendiamin in der erforderlichen Reinheit herzustellen, ist nach den bekannten Verfahren ein hoher Energieeinsatz erforderlich.

Es stellte sich die Aufgabe, ein verbessertes, energieeffizientes Verfahren zur Bereitstellung von Hexamethylendiamin hoher Reinheit zu finden.

Diese Aufgabe wird erfindungsgemäß gelöst durch ein Verfahren zur Abtrennung von leichtsiedenden Komponenten aus einem Hexamethylendiamin enthaltenden Gemisch gemäß Anspruch 1. Vorteilhafte Ausgestaltungen und Weiterbildungen der Erfindung sind in den abhängigen Ansprüchen angegeben.

Als leichtsiedende Komponenten werden im Rahmen der vorliegenden Erfindung solche Komponenten des Stoffgemisches bezeichnet, deren Siedepunkt bei Normaldruck niedriger ist als der Siedepunkt von Hexamethylendiamin.

Das erfindungsgemäße Verfahren wird in einer Destillationsvorrichtung durchgeführt, die einen Verstärkungsteil und einen Abtriebsteil umfasst. Das Hexamethylendiamin und abzutrennende leichtsiedende Komponenten enthaltende Gemisch wird als Feedstrom der Destillationsvorrichtung zugeführt. Am Kopf des Verstärkungsteils wird ein an leichtsiedenden Komponenten angereicherter Kopfstrom abgezogen, am Sumpf des Abtriebsteils wird ein um leichtsiedende Komponenten abgereicherter Sumpfstrom abgezogen. Ein Teilstrom dieses Sumpfstroms wird einem Sumpfverdampfer zugeführt, dort zumindest teilweise verdampft und in den Abtriebsteil zurückgeführt. Erfindungsgemäß wird weiterhin aus der Destillationsvorrichtung ein dampfförmiger Seitenstrom abgezogen und einem Verdichter zugeführt. Der verdichtete Strom wird als Heizmedium zum Sumpfverdampfer geleitet, wo er durch Abgabe von Wärme den Teilstrom aus dem Sumpf zumindest teilweise verdampft. Der verdichtete Seitenstrom kühlt sich dabei ab, wobei er zumindest teilweise kondensiert, und wird nach Austritt aus dem Sumpfverdampfer wieder der Destillationsvorrichtung zugeführt.

Die Destillationsvorrichtung kann unterschiedlich ausgestaltet sein. In einer bevorzugten Ausgestaltung handelt es sich bei der Destillationsvorrichtung um eine Kolonne, deren oberer Abschnitt vom Kopf bis zum Feedzulauf als Verstärkungsteil und deren unterer Abschnitt vom Feedzulauf bis zum Sumpf als Abtriebsteil fungiert.

Es wurde gefunden, dass es aus thermodynamischer Sicht günstig ist, die Zuführung des Feedstroms und den Abzug des Seitenstroms auf nahe beieinander liegenden Stufen der Destillationsvorrichtung vorzusehen. In einer bevorzugten Ausführungsform liegen die Zuführung des Feedstroms und der Abzug des Seitenstroms nicht mehr als drei theoretische Trennstufen auseinander. Besonders vorteilhaft aus apparatetechnischer Sicht ist die Anordnung der Zu-und Abfuhrleitungen an derselben Stelle im Hinblick auf die Höhe der Destillationsvorrichtung.

Weiterhin wurde gefunden, dass es ebenso vorteilhaft ist, auch die Zuführung des Seitenstroms nach Austritt aus dem Sumpfverdampfer in die Destillationsvorrichtung in der Nähe des Feedzulaufs und des dampfförmigen Seitenabzugs vorzusehen. In einer weiterhin bevorzugten Ausführungsform weicht die Zuführung des zumindest teilweise kondensierten Seitenstroms nach Austritt aus dem Sumpfverdampfer um nicht mehr als drei theoretische Trennstufen von der Zuführung des Feedstroms und dem Abzug des Seitenstroms ab. Besonders bevorzugt erfolgt sie auf derselben theoretischen Trennstufe wie der dampfförmige Seitenabzug.

In einer weiteren bevorzugten Ausgestaltung der Destillationsvorrichtung sind der Verstärkungsteil und der Abtriebsteil als separate Kolonnen ausgeführt, wobei der Sumpf des Verstärkungsteils mit dem Kopf des Abtriebsteils über mindestens eine Dampfleitung und mindestens eine Flüssigkeitsleitung verbunden ist. Der Feedstrom wird vorzugsweise am Kopf des Abtriebsteils zugeführt. Besonders bevorzugt wird bei dieser Konfiguration der Seitenstrom am Kopf des Abtriebsteils abgezogen und nach Durchlaufen des Verdichters sowie des Sumpfverdampfers dem Kopf des Abtriebsteils wieder zugeführt.

Bei dem erfindungsgemäßen Verfahren wird der Energiegehalt des aus der Destillationsvorrichtung abgezogenen dampfförmigen Seitenstroms durch dessen Kondensation genutzt, um einen Teilstrom aus dem Sumpf des Abtriebsteils zu verdampfen. In einer besonders bevorzugten Ausgestaltung des Verfahrens erfolgt die Verdampfung des Teilstroms ausschließlich durch Wärmeaustausch mit dem verdichteten Seitenstrom. Diese Wärmeintegration bewirkt gegenüber den bekannten Verfahren eine deutliche Reduzierung der Energiemenge, die für die Abtrennung von leichtsiedenden Komponenten aus dem Hexamethylendiamin enthaltenden Gemisch erforderlich ist.

Das Konzept der Wärmeintegration bei Destillationskolonnen ist seit längerem bekannt. So wird beispielsweise in dem Standardwerk "Perry's Chemical Engineer's Handbook, McGraw-Hill, 7. Auflage, Seiten 13-5 bis 13-8" eine Übersicht über den Stand der Technik der energieeffizienten Destillation gegeben. Es werden Konzepte mit Zwischenkühlern und Zwischenverdampfern ebenso vorgestellt wie Wärmepumpen, die den Energiegehalt des Kopfstroms oder des Sumpfstroms aus einer Kolonne zur Wärmeintegration nutzen. Wie unten stehend anhand eines Beispiels gezeigt wird, lässt sich bereits mit den bekannten Konzepten gegenüber der herkömmlichen Destillation eine Energieersparnis erzielen. Wie gezeigt wird, bietet das neu vorgeschlagene erfindungsgemäße Verfahren, bei dem ein Seitenstrom zur Wärmeintegration verwendet wird, eine nochmals deutlich weitergehende Energieeffizienz.

Als Einbauten zur Stofftrennung in den Kolonnen werden bevorzugt solche gewählt, die einen geringen Druckverlust aufweisen. Der Druckverlust sollte vorzugsweise höchstens 1,5 mbar, insbesondere höchstens 1 mbar pro theoretische Trennstufe aufweisen. Bevorzugt werden Packungen eingesetzt, besonders bevorzugt geordnete Packungen wie Metallblechpackungen oder Drahtgewebepackungen.

Weiterhin bevorzugt sind Destillationsvorrichtungen, bei denen der Innendurchmesser der Destillationsvorrichtung oberhalb des dampfförmigen Abzugs geringer ist als der Innendurchmesser der Destillationsvorrichtung unterhalb des dampfförmigen Abzugs. Dadurch, dass aufgrund der erfindungsgemäßen Konfiguration ein Großteil des Dampfes in dem Teil der Destillationsvorrichtung unterhalb des dampfförmigen Seitenabzugs zirkuliert, sind die Gasbelastungen in dem oberen und unteren Kolonnenteil unterschiedlich. Eine Ausführungsform mit unterschiedlichen Innendurchmessern oberhalb und unterhalb des Seitenabzugs berücksichtigt dies und trägt zur Einsparung von Investitionskosten bei.

In einer bevorzugten Ausführungsform der Erfindung wird der Feedstrom der Destillationsvorrichtung dampfförmig zugeführt. In einem Gesamtverfahren zur Bereitstellung von reinem Hexamethylendiamin der eingangs erläuterten Art entstammt der Feedstrom zur erfindungsgemä-βen Destillationsvorrichtung dem Kopfstrom eines vorangehenden Trennschrittes. Durch eine dampfförmige Zufuhr des Feedstroms kann auf eine Kondensation des Kopfstroms der vorhergehenden Stufe verzichtet werden. Dadurch wird im Gesamtverfahren ein Apparat weniger benötigt, was wirtschaftlich vorteilhaft ist.

Bei einer weiteren bevorzugten Ausführungsform wird der aus der Destillationsvorrichtung abgezogene Seitenstrom vor dem Eintritt in den Verdichter durch einen Wärmetauscher geleitet. In dem Wärmetauscher wird die Temperatur des Seitenstroms mindestens so weit erhöht, dass eine Kondensation des Seitenstroms während des Verdichtens ausgeschlossen ist. Durch diese Überhitzung des dampfförmigen Seitenstroms wird der Gefahr vorgebeugt, dass beim Verdichten Flüssigkeitströpfchen entstehen, die den Verdichter schädigen könnten.

Es hat sich als vorteilhaft erwiesen, zumindest einen Teil des zugeführten dampfförmigen Feedstroms ebenfalls zu verdichten und zur Beheizen des Sumpfverdampfers zu verwenden. In einer bevorzugten Ausgestaltung der Erfindung wird daher der Feedstrom zumindest teilweise mit dem Seitenstrom zusammengeführt, bevor der daraus resultierende Strom in den Verdichter oder, sofern vorhanden, in den Wärmetauscher vor dem Verdichter geleitet wird. Die Zusammenführung kann innerhalb der Destillationsvorrichtung erfolgen, indem beispielsweise der dampfförmige Feedzulauf auf derselben Stufe vorgesehen wird wie der dampfförmige Seitenabzug. Einbauten auf der entsprechenden Stufe wie Leitbleche zur Beeinflussung der Dampfströmung können vorgesehen werden, um den Anteil des Feedstroms einzustellen, der dem Seitenstrom zugefügt wird. Bevorzugt wird der Feedstrom vollständig dem Seitenstrom zugeführt. Eine apparativ einfache Umsetzung besteht beispielsweise darin, die Feedleitung außerhalb der Destillationsvorrichtung in die Leitung des Seitenabzugs münden zu lassen. Verfahrenstechnisch bietet diese Variante den Vorteil, dass sich die beiden Dampfströme schnell und gut durchmischen.

Bevorzugt wird die Destillationsvorrichtung bei einem Absolutdruck am Kopf des Verstärkungsteils von weniger als 300 mbar, besonders bevorzugt von 80 bis 225 mbar, insbesondere von 90 bis 110 mbar betrieben. Der Druckverlust vom Kopf des Verstärkungsteils bis zum Sumpf des Abtriebsteils beträgt bevorzugt von 70 bis 300 mbar, besonders bevorzugt von 70 bis 120 mbar. In dieser bevorzugten Ausgestaltung übersteigt der Absolutdruck im Sumpf des Abtriebsteils den Wert von 400 mbar nicht. Durch eine Begrenzung des Drucks im Sumpf sowie der Wahl des Kopfdrucks in den bevorzugten Bereichen wird die Abtrennung der leichtsiedenden Komponenten aus dem Wertprodukt Hexamethylendiamin begünstigt.

Zur Sicherstellung der Qualität des über Sumpf der Destillationsvorrichtung abgezogenen Hexamethylendiamin-reichen Produktes werden die Betriebsparameter des erfindungsgemäßen Verfahrens wie Rücklaufverhältnis, Kondensationsleistung am Kopf des Verstärkungsteils und Verdichtungsverhältnis vorzugsweise so gewählt, dass die Gewichtsanteile der leichtsiedenden Komponenten im Sumpf des Abtriebsteils jeweils von 0 bis 100 ppm, besonders bevorzugt von 0 bis 50 ppm, insbesondere von 0 bis 25 ppm betragen.

Das erfindungsgemäße Verfahren ermöglicht es, Hexamethylendiamin hoher Reinheit bereitzustellen, wobei im Vergleich zu bekannten Verfahren eine deutliche Einsparung an extern aufzuwendender Energie erzielt werden kann.

Anhand der Zeichnungen wird im Folgenden die Erfindung weiter erläutert, wobei die Zeichnungen als Prinzipdarstellungen zu verstehen sind. Sie stellen keine Beschränkung der Erfindung, beispielsweise im Hinblick auf konkrete Abmessungen oder Ausgestaltungsvarianten von Bauteilen dar. Es zeigen:
- Fig. 1:: Verfahrensschema der konventionellen Destillation gemäß Stand der Technik
- Fig. 2:: Verfahrensschema einer bekannten Wärmeintegration mit Brüdenverdichtung
- Fig. 3:: Verfahrensschema einer ersten erfindungsgemäßen Ausführungsform
- Fig. 4:: Verfahrensschema einer zweiten erfindungsgemäßen Ausführungsform

Sofern nicht anderweitig beschrieben sind Werte von Konzentrationen in Gewichtsanteilen und Drücke als Absolutdrücke angegeben. Die im Folgenden näher ausgeführten Konfigurationen wurden mit einem Simulationsprogramm ausgelegt und berechnet.

### Vergleichsbeispiele

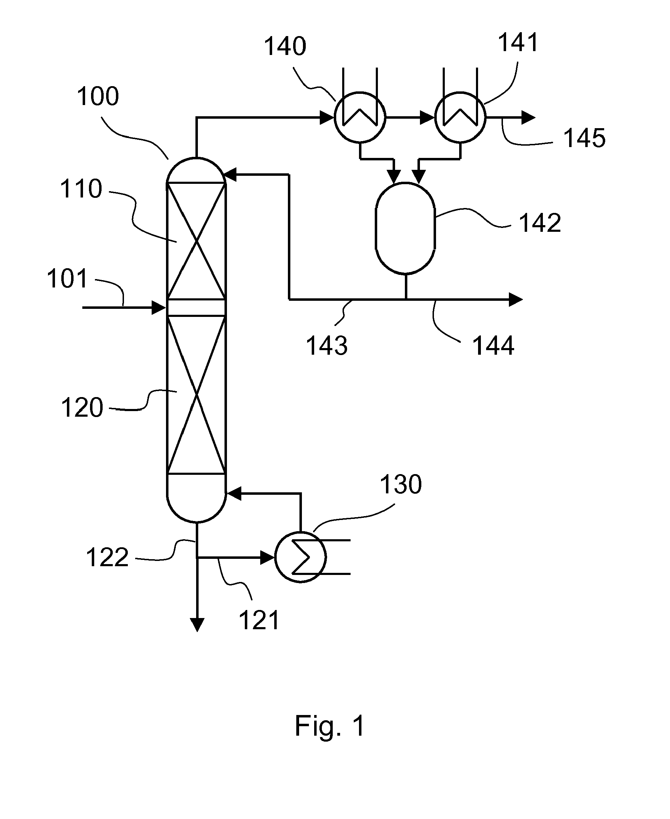

Fig. 1 zeigt schematisch eine Kolonne als Destillationsvorrichtung 100, wie sie aus dem Stand der Technik bekannt ist. Als Trenneinbauten im Verstärkungsteil 110 und Abtriebsteil 120 werden strukturierte Packungen verwendet mit insgesamt 124 theoretischen Trennstufen. Zwischen Verstärkungsteil 110 und Abtriebsteil 120 wird über den Feedzulauf 101 ein dampfförmiges Gemisch zugeführt, das als leichtsiedende Komponenten Wasser (530 ppm), Hexamethylenimin (110 ppm), Diaminocyclohexan (4560 ppm), Diaminomethylpentan (90 ppm), und Aminomethylcyclopentylamin (330 ppm) enthält. Die Hauptkomponente mit 99,42% ist Hexamethylendiamin. Daneben sind noch geringe Mengen an Aminocapronitril (40 ppm) und Schwersiedern enthalten. Die Kolonne 100 wird bei einem Kopfdruck von 130 mbar und einem Rücklaufverhältnis von 927 betrieben. Als Rücklaufverhältnis wird der Quotient aus dem Mengenstrom des Rücklaufs 143 und dem abgezogenen Destillatstrom 144 bezeichnet. Die Temperatur im Kopf der Kolonne beträgt 124,5°C, die Temperatur im Sumpf 147,5°C.

Am Kopf der Kolonne 100 wird ein dampfförmiger Kopfstrom abgezogen, in dem die leichtsiedenden Komponenten angereichert sind. Dieser Kopfstrom wird zunächst in einem Hauptkondensator 140 teilweise kondensiert. Der nicht kondensierte Anteil des Dampfes wird einem Nachkondensator 141 zugeführt, wo bei einer tieferen Temperatur ein weiterer Anteil des Dampfstromes kondensiert. Der verbleibende Anteil des Dampfstromes, der auch im Nachkondensator nicht vollständig kondensiert, wird über die Inertenausschleusung 145 aus dem System entfernt. Die während der Kondensation entstandene Flüssigkeit aus beiden Kondensatoren wird in einen Kondensatsammelbehälter 142 geleitet. Von diesem wird ein Teil als Rücklauf 143 auf den Kopf der Kolonne zurückgefahren, der übrige Teil wird als Destillatstrom 144 abgeführt. Für die Kondensation wird in Summe eine spezifische Kühlenergie von 803,5 kWh pro Tonne Produkt aufgewandt. Unter dem Produkt wird hier und im Folgenden das aus dem Sumpf des Abtriebsteils gewonnene und aus der Destillationsvorrichtung ausgeschleuste Hexamethylendiamin-Wertprodukt verstanden.

Aus dem Sumpf der Kolonne 100 wird ein Sumpfstrom 122 abgezogen, der um leichtsiedende Komponenten abgereichert ist. Hexamethylendiamin wird über Sumpf in hoher Reinheit gewonnen, der Anteil an Aminomethylcyclopentylamin beträgt lediglich 20 ppm, der Anteil an Diaminocyclohexan weniger als 5 ppm. Von diesem Sumpfstrom 122 wird ein Teilstrom 121 einem Sumpfverdampfer 130 zugeführt, wo er durch externe Wärmezufuhr zumindest teilweise verdampft und dem Abtriebsteil 120 der Kolonne wieder zugeführt wird. Als Sumpfverdampfer wird ein Fallfilmverdampfer eingesetzt, der mit Wasserdampf beheizt ist. Die zugeführte spezifische Wärmemenge beträgt 680,5 kWh pro Tonne Produkt. Das "Produkt" entspricht im Beispiel gemäß Fig. 1 dem Strom, der von dem Sumpfstrom 122 übrig bleibt, nachdem der Teilstrom 121 abgezogen wurde.

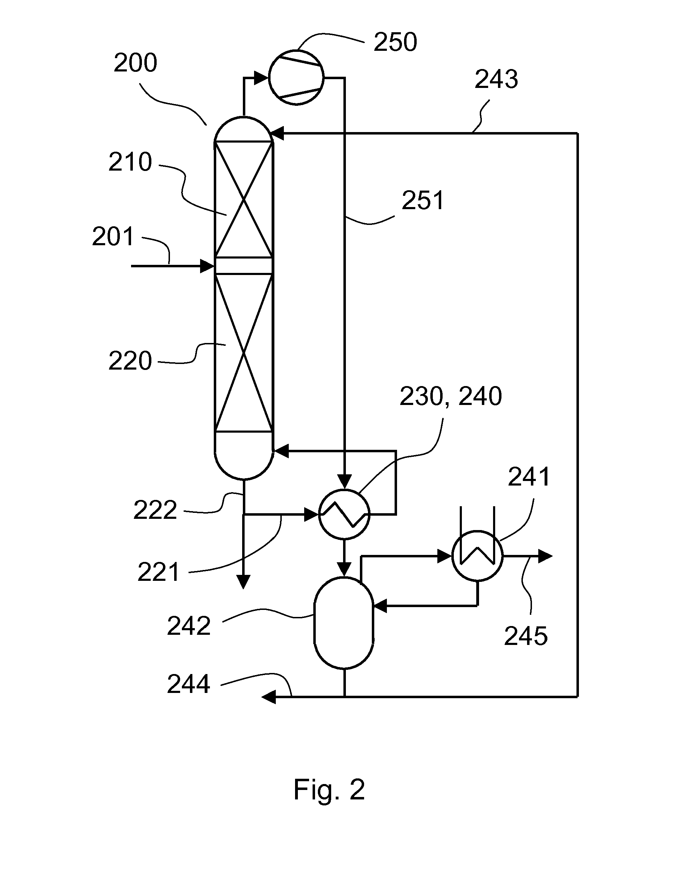

In Fig. 2 ist schematisch eine Kolonne als Destillationsvorrichtung 200 dargestellt, bei der eine Wärmeintegration mit Brüdenverdichtung gemäß dem Stand der Technik verwirklicht ist. Um dieses Verfahren mit der konventionellen Destillation gemäß Fig. 1 vergleichen zu können, sind Verstärkungsteil 210 und Abtriebsteil 220 ebenfalls mit strukturierten Packungen als Trenneinbauten mit insgesamt 124 theoretischen Trennstufen ausgestattet. Die Zusammensetzung des Feedstrom 201 ist identisch wie in obigem Fall, und die Kolonne 200 wird ebenfalls bei einem Kopfdruck von 130 mbar betrieben. Um dieselben Reinheiten im Sumpfstrom 222 zu erzielen, ist ein Rücklaufverhältnis von 759 erforderlich. Das Rücklaufverhältnis ist wie oben als Quotient des Rücklaufstroms 243 zum abgezogenen Destillatstrom 245 definiert. Die Temperatur im Kopf beträgt 124,5°C, im Sumpf 147,5°C.

Der dampfförmige Kopfstrom wird zunächst einem Verdichter 250 zugeführt, in dem er auf einen höheren Druck und eine höhere Temperatur gebracht wird. Zur Verdichtung wird eine spezifische Energie von 207,1 kWh pro Tonne Produkt aufgewandt. Der verdichtete Dampfstrom wird dem Hauptkondensator 240 zugeführt, der gleichzeitig als Sumpfverdampfer 230 dient. In dem Sumpfverdampfer kondensiert der verdichtete Brüdenstrom teilweise und verdampft dadurch einen Teilstrom 221 aus dem Sumpfstrom 222. Der zumindest teilweise verdampfte Teilstrom wird dem Abtriebsteil 220 der Kolonne wieder zugeführt. Die Temperatur des Dampfstroms beim Austritt aus dem Verdichter 250 ist so gewählt, dass sich in dem Wärmetauscher 230/240 eine logarithmische Temperaturdifferenz von 8 Kelvin einstellt. Die im Wärmetauscher 230/240 übertragene spezifische Wärme beträgt 676,7 kWh pro Tonne Produkt.

Der nicht kondensierte Anteil des verdichteten Brüdenstroms wird einem Nachkondensator 241 zugeleitet, wo ein weiterer Anteil kondensiert wird. Der auch im Nachkondensator nicht kondensierte Anteil des Dampfes wird über eine Inertenausschleusung 245 aus dem System entfernt. Die kondensierte Flüssigkeit wird in einen Kondensatsammelbehälter 242 geleitet und wird von dort teilweise als Rücklaufstrom 243 auf den Kopf der Kolonne zurückgeführt. Der übrige Teil wird als Destillatstrom 244 ausgeleitet. Für die Kondensation im Nachkondensator 241 wird eine externe Kühlenergie von 335,7 kWh pro Tonne Produkt aufgewandt.

### Erfindungsgemäßes Beispiel

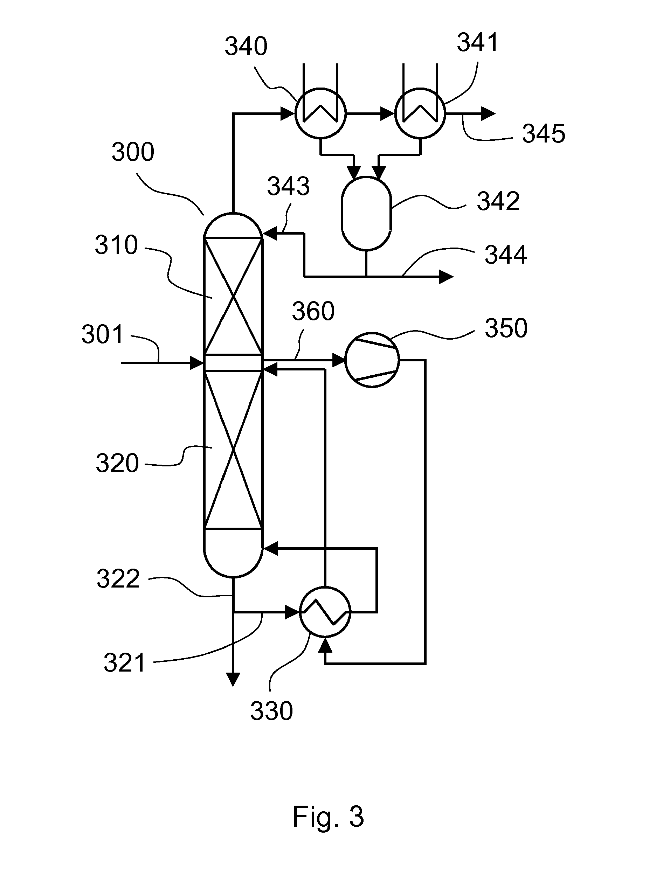

Fig. 3 zeigt schematisch eine erste Ausführungsform der Erfindung. Einer Kolonne als Destillationsvorrichtung 300 wird ein gasförmiger Feedstrom 301 zugeführt, der dieselbe Zusammensetzung aufweist wie in den oben ausgeführten Vergleichsbeispielen. Verstärkungsteil 310 und Abtriebsteil 320 der Kolonne sind mit strukturierten Packungen als Trenneinbauten mit insgesamt 124 theoretischen Trennstufen bestückt. Der dampfförmige Kopfstrom wird einem Hauptkondensator 340 und einem Nachkondensator 341 zugeführt. Der nicht kondensierte Anteil wird als Inertenstrom 345 ausgeschleust, die kondensierte Flüssigkeit in einen Kondensatsammelbehälter 342 geleitet. Aus dem Behälter 342 wird ein Teil der Flüssigkeit als Rücklauf 343 auf den Kopf der Kolonne zurückgeführt, der übrige Teil wird als Destillatstrom 344 abgeführt.

Aus der Destillationsvorrichtung 300 wird ein dampfförmiger Seitenstrom 360 abgezogen und einem Verdichter 350 zugeführt. Für das konkrete Beispiel wurde der Feedboden als günstigster Ort des Abzugs gefunden. Der Seitenstrom 360 wird durch die Verdichtung auf einen höheren Druck und eine höhere Temperatur gebracht und wird dem Sumpfverdampfer 330 zugeführt. Dort kondensiert der verdichtete Seitenstrom zumindest teilweise. Die frei werdende Kondensationswärme wird im Sumpfverdampfer 330 an einen Teilstrom 321 des Sumpfstromes 322, der aus dem Kolonnensumpf abgezogen wird, übertragen, wodurch dieser zumindest teilweise verdampft. Die Temperatur des Dampfstroms beim Austritt aus dem Verdichter 350 ist so gewählt, dass sich in dem Sumpfverdampfer 330 eine logarithmische Temperaturdifferenz von 8 Kelvin einstellt. Der Teilstrom wird nach Austritt aus dem Sumpfverdampfer 330 dem Abtriebsteil 320 der Kolonne wieder zugeführt. Die im Sumpfverdampfer übertragene spezifische Wärme beträgt 861,6 kWh pro Tonne Produkt.

Die Wärmeintegration unter Verwendung eines Seitenstromes 360 aus der Kolonne hat gegenüber dem oben beschriebenen konventionellen Konzept mehrere Vorteile. Um dieselbe Reinheit des Sumpfstromes zu erhalten, genügt bei dem erfindungsgemäßen Verfahren ein Rücklaufverhältnis von 222. Dadurch wird der Verstärkungsteil hinsichtlich der Flüssigkeitsbelastung deutlich entlastet. Dies ermöglicht es, den Verstärkungsteil im Durchmesser kleiner auszulegen als den Abtriebsteil, wodurch Investitionskosten eingespart werden können. Auch die erforderliche Energiezufuhr aus externen Quellen wird signifikant verringert. Für beide Kondensatoren 340, 341 zusammen ist eine spezifische Kühlenergie von 188,7 kWh pro Tonne Produkt erforderlich, die zum Betrieb des Verdichters aufzuwendende spezifische Energie beträgt 73,0 kWh pro Tonne Produkt.

Die nachfolgende Tabelle stellt den Energiebedarf der beiden konventionellen und des erfindungsgemäßen Verfahrens gegenüber.

| Energie in kWh pro Tonne Produkt | Konventionelle Destillation | Konventionelle Wärmeintegration | Erfindung |
|---|---|---|---|
| Externe Energie für Verdampfung | 680,5 | 0 | 0 |
| Externe Energie für Kondensation | 803,5 | 335,7 | 188,7 |
| Externe Energie für Verdichter | 0 | 207,1 | 73,0 |
| Summe | 1484,0 | 542,8 | 261,7 |

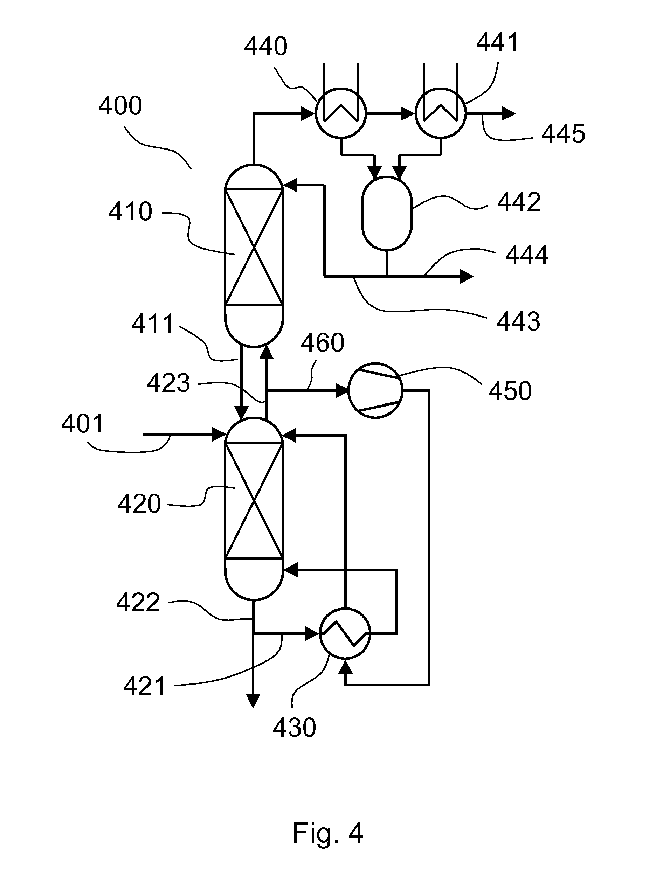

Fig. 4 zeigt eine weitere bevorzugte Ausführungsform des erfindungsgemäßen Verfahrens. In diesem Fall umfasst die Destillationsvorrichtung 400 zwei separate Kolonnen, die als Verstärkungsteil 410 und Abtriebsteil 420 fungieren. Die beiden Kolonnen sind durch eine Flüssigkeitsleitung 411 und eine Dampfleitung 423 verbunden. Ein gasförmiger Feedstrom 401 wird dem Kopf des Abtriebsteils 420 zugeführt. Der den Kopf des Verstärkungsteils 410 verlassende Kopfstrom wird in einem Hauptkondensator 440 und einem Nachkondensator 441 größtenteils kondensiert. Der nicht kondensierte Teil wird als Inertenstrom 445 ausgeschleust. Die kondensierte Flüssigkeit fließt in einen Kondensatsammelbehälter 442, von wo aus sie zum Teil als Rücklaufstrom 443 auf den Kopf des Verstärkungsteils zurückgeführt wird. Der übrige Anteil verlässt als Destillatstrom 444 das System.

Am Sumpf des Abtriebsteils 420 wird ein Sumpfstrom 422 abgezogen, von dem ein Teilstrom 421 dem Sumpfverdampfer 430 zugeführt wird. Dort wird der Teilstrom 421 zumindest teilweise verdampft und dem Sumpf des Abtriebsteils 420 wieder zugeführt.

Aus der Dampfleitung 423, die den Abtriebsteil 420 mit dem Verstärkungsteil 410 verbindet, wird ein dampfförmiger Seitenstrom 460 abgezweigt und einem Verdichter 450 zugeführt. Der verdichtete Strom wird durch den Sumpfverdampfer 430 geleitet, wo er zumindest teilweise kondensiert und Wärme an den Teilstrom 421 aus dem Sumpf des Abtriebsteils abgibt und diesen verdampft.

## Patentansprüche

1. Verfahren zur Abtrennung von leichtsiedenden Komponenten aus einem Hexamethylendiamin enthaltenden Gemisch, wobei das Gemisch als Feedstrom (301, 401) einer Destillationsvorrichtung (300, 400) zugeführt wird, die einen Verstärkungsteil (310, 410) und einen Abtriebsteil (320, 420) umfasst, am Kopf des Verstärkungsteils ein an leichtsiedenden Komponenten angereicherter Kopfstrom abgezogen wird, am Sumpf des Abtriebsteils ein um leichtsiedende Komponenten abgereicherter Sumpfstrom (322, 422) abgezogen wird, von dem ein Teilstrom (321, 421) einem Sumpfverdampfer (330, 430) zugeführt, dort zumindest teilweise verdampft und in den Abtriebsteil zurückgeführt wird, **dadurch gekennzeichnet, dass** aus der Destillationsvorrichtung ein dampfförmiger Seitenstrom (360, 460) abgezogen und einem Verdichter (350, 450) zugeführt wird, der verdichtete Strom als Heizmedium zum Sumpfverdampfer (330, 430) geleitet wird, wo er durch Abgabe von Wärme den Teilstrom (321, 421) aus dem Sumpf zumindest teilweise verdampft, und der zumindest teilweise kondensierte Seitenstrom nach Austritt aus dem Sumpfverdampfer wieder der Destillationsvorrichtung zugeführt wird.

2. Verfahren nach Anspruch 1, wobei der Feedstrom (301, 401) dampfförmig zugeführt wird.

3. Verfahren nach Anspruch 1 oder 2, wobei der Seitenstrom (360, 460) vor dem Eintritt in den Verdichter (350, 450) durch einen Wärmetauscher geleitet wird, in dem seine Temperatur mindestens so weit erhöht wird, dass eine Kondensation des Seitenstroms während des Verdichtens ausgeschlossen ist.

4. Verfahren nach einem der Ansprüche 1 bis 3, wobei die Verdampfung des Teilstroms (321, 421) aus dem Sumpf des Abtriebsteils ausschließlich durch Wärmeaustausch mit dem verdichteten Seitenstrom erfolgt.

5. Verfahren nach einem der Ansprüche 1 bis 4, wobei die Gewichtsanteile der leichtsiedenden Komponenten im Sumpf des Abtriebsteils (320, 420) jeweils von 0 bis 100 ppm, bevorzugt von 0 bis 50 ppm, insbesondere von 0 bis 25 ppm betragen.

6. Verfahren nach einem der Ansprüche 1 bis 5, wobei der Absolutdruck am Kopf des Verstärkungsteils (310, 410) weniger als 300 mbar, bevorzugt von 80 bis 225 mbar, insbesondere von 90 bis 110 mbar beträgt, und der Druckverlust vom Kopf des Verstärkungsteils bis zum Sumpf des Abtriebsteils (320, 420) von 70 bis 300 mbar, insbesondere von 70 bis 120 mbar beträgt, wobei der Absolutdruck im Sumpf des Abtriebsteils 400 mbar nicht übersteigt.

7. Verfahren nach einem der Ansprüche 1 bis 6, wobei es sich bei der Destillationsvorrichtung (300) um eine Kolonne handelt, deren oberer Abschnitt vom Kopf bis zum Feedzulauf als Verstärkungsteil (310) und deren unterer Abschnitt vom Feedzulauf bis zum Sumpf als Abtriebsteil (320) fungiert.

8. Verfahren nach einem der Ansprüche 1 bis 6, wobei der Verstärkungsteil (410) und der Abtriebsteil (420) als separate Kolonnen ausgeführt sind, und wobei der Sumpf des Verstärkungsteils mit dem Kopf des Abtriebsteils über mindestens eine Dampfleitung (423) und mindestens eine Flüssigkeitsleitung (411) verbunden ist.

9. Verfahren nach Anspruch 8, wobei der Seitenstrom (460) am Kopf des Abtriebsteils (420) abgezogen und nach Durchlaufen des Verdichters (450) und Sumpfverdampfers (430) dem Kopf des Abtriebsteils wieder zugeführt wird.

10. Verfahren nach einem der Ansprüche 1 bis 9, wobei die Zuführung des Feedstroms (301, 401) und der Abzug des Seitenstroms (360, 460) nicht mehr als drei theoretische Trennstufen auseinander liegen.

11. Verfahren nach Anspruch 10, wobei die Zuführung des zumindest teilweise kondensierten Seitenstroms nach Austritt aus dem Sumpfverdampfer (330, 430) um nicht mehr als drei theoretische Trennstufen von der Zuführung des Feedstroms und dem Abzug des Seitenstroms abweicht, insbesondere auf derselben theoretischen Trennstufe wie der Abzug des Seitenstroms erfolgt.

12. Verfahren nach einem der Ansprüche 2 bis 11, wobei der Feedstrom (301, 401) zumindest teilweise mit dem Seitenstrom (360, 460) zusammengeführt wird, bevor der daraus resultierende Strom in den Verdichter (350, 450) oder gegebenenfalls den Wärmetauscher vor dem Verdichter geleitet wird.
